# EUROPEAN PATENT APPLICATION

(11) **EP 4 438 047 A1**
(43) Date of publication of application: **02.10.2024**
(21) Application number: 23164382.6
(22) Date of filing: 27.03.2023
(51) Int. Cl.: A61K 31/7084, A61P 31/14, A61P 43/00

(54) **BIOLOGICAL RESPONSE MODIFIERS FOR THE TREATMENT OF SUBJECTS WITH UNDERPERFORMING IMMUNE SYSTEMS AND COMPOSITIONS THEREOF**

(71) Applicant: Helmholtz-Zentrum für Infektionsforschung GmbH, 38124 Braunschweig (DE); Center for Genetic Engineering and Biotechnology ( CIGB), La Habana 10600 (CU)
(72) Inventor: Aguilar Rubido, Julio Cesar, 10600 Havanna (CU); Guzmán, Carlos A., 38124 Braunschweig (DE); Freyre Almeida, Freya Milagros, 10600 Havanna (CU); Guillen Nieto, Gerardo Enrique, 10600 Havanna (CU); Ebensen, Thomas, 38124 Braunschweig (DE); Riese, Peggy, 38124 Braunschweig (DE); Schulze, Kai, 38124 Braunschweig (DE); Trittel, Stephanie, 38124 Braunschweig (DE)
(74) Representative: Kröncke, Rolf

(57) **Abstract**

In the present invention, the cyclic-di-nucleotides or their compositions are used as biological response modifiers to improve the immune response of subject with underperforming immune systems with low/non-responsiveness to vaccine antigens or underperforming immune response to pathogens. The formulations of the present invention are capable of modifying the biological response in the way of abrogating the non-responsiveness resulting in the development of a high avidity immune response preventing the disease caused by infectious agents, in particular from viruses, with the capacity to evade the mechanisms of immune surveillance and consequently suppress the induction of a high avidity and effective adaptive response. The present invention also comprises the formulations of cyclic di-nucleotides, which may be administered alone, in combination with other biological response modifiers or in vaccine formulations with antigens. The proposed invention also includes the method of preventing or treating acute respiratory infections, preventing the severity of the disease and blocking transmission by inducing an immune response at the entry site (nasopharyngeal mucosa), preventing or reducing person-to-person pathogen/virus transmission in pre-emptive vaccine formulations as well as in formulations used for pre-/post exposure prophylaxis of acute respiratory infections.

## Description

### Field of the invention

The present invention relates to the field of vaccinology, specifically to the area of biological response modifiers with capacity to improve the immune response of low/non-responsive subjects to vaccine antigens or to pathogen infections. The present invention comprises the formulations of cyclic di-nucleotides, which may be administered alone, in combination with other biological response modifiers or in vaccine formulations with specific antigens. The proposed invention also includes the method of preventing or treating acute respiratory infections, preventing the severity of the disease and blocking transmission by inducing an immune response at the entry site (nasopharyngeal mucosa), preventing or reducing person-to-person pathogen/virus transmission in pre-emptive vaccine formulations as well as in formulations used for pre-/post-exposure prophylaxis of acute respiratory and febrile infections.

### Previous Art

The avidity of antibodies denotes the strength of binding to its target epitope and reflects the best fit between IgG and epitope. The emergence of the SARS-CoV-2 coronavirus disease 2019 (COVID-19) pandemic have produced a rapid development of vaccines and therapeutics. The interaction of SARS-CoV-2 S protein with the host cells is a crucial component for both continuing vaccine development.

The efficient rupture of the strong interaction established between SARS-CoV-2 RBD and human ACE2 protein has been proposed to require the high-binding affinity antibodies toward multiples sites of RBD (at least two molecular epitopes), which highlights the importance of functional affinity in blockage SARS-CoV-2 infection (Khatri, 2020). Other viral infections such as, cytomegalovirus (CMV) , Dengue and Vesicular Stomatitis Virus require antibodies with high avidity, instead of high titers, to confer effective protection. A similar result was also reported in a study evaluating the immune response to malaria's vaccine, Dobaño C, et. al., Nature communications 2019;10(1),1-13..

The interaction between the receptor-binding domain of SARS-CoV-2 spike protein and ACE2 on target cells is the initial step in infection. The binding between the RBD of SARS-CoV-2 and ACE2 is characterized by high affinity, Khatri I, et. Al., Front. Immunol. 2020;11:570018. Therefore, Khatri and colleagues proposed that efficient neutralization of SARS-CoV-2 would require antibodies of high avidity, adding to the classical concept of neutralizing antibodies the additional requirement for high affinity/avidity.

The association between antibody neutralizing titers and antibody avidity contribute to the understanding of humoral responses to SARS-CoV-2 (Benner SE, et.al., J Infect Dis. 2020 Nov 13;222(12):1974-1984.). As described for other pathogens, antibody avidity increases over duration of infection, in SARS-CoV-2 the same is observed, once low antibody avidity is showed during early infection and the avidity increase after 3 weeks of symptom onset in patients recovered from COVID-19. However, the antibody response towards infection with SARS-CoV-2 and seasonal coronaviruses is characterized by incomplete avidity maturation, as has been well characterized by several groups.

The analysis of outpatients with Covid-19 and SARS-CoV-2 infection confirmed by positive polymerase chain reaction (PCR) led, however, to a rather unexpected result, Bauer G, et.al., J Med Virol. 2021;93:3092-3104. In contrast to the regular interaction between the humoral immune system and viruses, the avidity of IgG towards SARS-CoV-2 antigens seemed to remain low in the majority of patients, even several months after the onset of clinical symptoms.

Waning IgG titers after SARS-CoV-2 infection, including for neutralizing antibodies, have also been shown by other groups. Compared to the Epstein-Barr virus (EBV) (a classical system for the application of avidity determination), SARS-CoV-2 infection is similar in low avidity during the first few weeks of infection, but completely different in avidity reached in past infection. Less than 15% of SARS-CoV-2 infected patients reached avidity indices >0.6 several months after infection, in contrast to more than 90% in the case of EBV. Similar high avidity indices have been reported for past infections with hepatitis A virus (HAV), hepatitis C virus (HCV), West Nile virus and other viruses.

Incomplete avidity maturation seems to be an essential strategy of coronaviruses in general. Incomplete avidity maturation was found for the serological response towards SARS-CoV-2 and the immune responses towards four seasonal coronaviruses (Bauer, 2021, see above; Friedhelm Struck, et.al., 2021 J Med Virol;93(12):6765-6777. doi: 10.1002/jmv.27270. Epub 2021 Aug 20. It is also known that the immune responses towards seasonal coronaviruses are characterized by a relatively rapid decline after an initial burst. It seems that these two characteristics of the immune response towards seasonal coronaviruses, i.e. declining antibody concentrations and frequent low avidity, are connected with the high probability of repeated waves of reinfections with these viruses. Recent data confirm that coronaviruses interfere with avidity maturation to ensure incomplete protection towards reinfection, allowing replication through repeated waves of infection in the host population. Thus, a valid approach would be to develop vaccines to induce a strong avidity antibody response.

The concept of a coronavirus immune evasion strategy that renders immune responses non-protective, combined with the biophysical data on the high affinity between SARS-CoV-2 RBD and its cellular receptor, make us propose a vaccination strategy which exploits certain qualities of the IgG in order to establish protective immunity. It has been considered that the vaccination programs should achieve (i) stimulation of sufficiently high and long-lasting IgG concentrations that specifically target viral structures relevant for binding to cellular receptors such as RBD; (ii) a complete avidity maturation of neutralizing IgG towards SARS-CoV-2; and (iii) that these antibodies should prevent the binding of the virus to the cells, ideally at mucosal compartments to prevent both local infection and spreading/transmission to susceptible hosts. Therefore, the suggested goal is that immunization should achieve a response that outcompetes the quality of the immune response reached after natural infection.

The development of novel compounds with the capacity of inducing high avidity antibodies represents a challenge in vaccinology. Different adjuvants enhance the immune response producing a strong antibody response or Th1 cellular response. However, the products with capacities as 'biological response modifiers' (BRMs) able to induce a strong and high avidity antibody response are limited. New formulations have been developed to solve this unmet need of current vaccines. For example, Poly(I:C) and CpG compounds adsorbed on aluminum hydroxide induce a strong immune response with a significant increase in antibody avidity. The formulation has different properties compared to the use of the compounds separately (Lu F, et.al., Vaccine. 2019;37(14):1945-1953).

Single intradermal or intramuscular inoculations of GM-CSF DNA with the DNA prime for a simian-human immunodeficiency virus (SHIV)-89.6 vaccine, which consists of DNA priming followed by modified vaccinia Ankara (MVA) boosting, increased protection of both the blood and intestines against the acute phase of an intrarectal SHIV-89.6P challenge. GM-CSF appeared to contribute to protection by enhancing two antibody responses: the avidity maturation of anti-Env IgG in blood and the presence of long lasting anti-viral IgA in rectal secretions. The avidity of anti-Env IgG showed strong correlations with protection both pre and post challenge. Animals with the highest avidity anti-Env Ab had 1000-fold reductions in peak viremia over those with the lowest avidity anti-Env Ab (Lai L, et.al., Virology. 2007;369(1):153-167).

TLR agonists and cytokines are involved in affinity maturation, however, these compounds are -as a rule- provided in combination with depot adjuvants to further improve this effect. However, in specific scenarios TLR agonists are unable to increase the avidity creating a confusing scenario that requires the optimization of the product case by case. Primary vaccination with TLR or oil-in-water-TLR adjuvants elicits high-avidity antibodies - as demonstrated for these "Adjuvant systems" (AS) combined with several antigens (e. g. Khurana, 2018) and the induced memory B cells undergo further maturation and differentiation upon antigen recall. Affinity maturation parameters appeared to differ when comparing with Alum. The avidity maturation appeared to be more strongly promoted by the AS formulations

A recent article published by Budroni et al., see above, and the team of experts from Glaxo-Smith-Kline specialized in the field of adjuvant development, recognized that the effects of adjuvant composition on durability and functionality of immune memory that can be boosted by antigen recall, remain under-investigated and that the differences in innate immune 'imprinting' between vaccine adjuvants may mediate dissimilar effects on the quantity/quality of persisting adaptive responses. In their studies, avidity maturation appeared to be more strongly promoted by their AS formulations when compared to Alum. Post-antigen recall, frequencies of subjects with high-avidity antibodies increased only markedly in the AS groups. Among the AS, total antibody responses were lowest for AS04. However, proportions of high-avidity antibodies were similar between groups, suggesting that monophosphoryl lipid A (MPL) in AS04 contributes to avidity maturation. Specific combinations of immunoenhancers in the AS, regardless of their individual nature, increase antibody persistence and avidity maturation. Nevertheless, the antibody avidity remains largely unexplored in current vaccinology and could be considered associated to some adjuvants and not to the others, independently of the intensity of the humoral immune response. In addition, as it is being explored by Budroni and colleagues, the formulation of adjuvants may induce specific effects that may be related to some specific compounds and not to others or to alum.

Regarding protection, recent studies confirmed that the level of antigen-antibody binding avidity could also correlate with protection. This has been demonstrated for the RTS, S malaria vaccine, amongst others, and for several monoclonal antibodies (McAb) treatments [Dobaño, 2019, see above]. Reversely, low-avidity antibodies have been associated with antibody-mediated disease enhancement following respiratory syncytial virus, dengue, or pandemic influenza vaccinations [Smatti MK, et.al., Front. Microbiol. 2018;9:2991].

Cyclic dinucleotide (CDNs) including cyclic di-adenosine monophosphate (cyclic di-AMP.; CDA), cyclic di-guanosine monophosphate (cyclic di-GMP; CDG), and cyclic GMP-AMP (cGAMP) are a class of bacterial and mammalian second messengers with potent immunological effector functions [Ebensen T, et. al.; Vaccine 2011;29(32),5210-5220]. CDNs can be used as adjuvants of protein subunit vaccines to trigger mucosal immunity to protect mice from respiratory bacterial infections in experimental animal models, such as *Mycobacterium tuberculosis,* anthrax, *Klebsiella pneumonia* and *Streptococcus pneumoniae, Acinetobacter baumannii,* and methicillin-resistant *Staphylococcus aureus.* CDNs also induce long-lasting CD8+ T cell immunity and exhibit a therapeutic effect against cancer in mice. Thus, CDNs generate long-lasting memory humoral and cellular responses in both the systemic and mucosal compartments [Ebensen, 2011, see above].

Superior to other vaccine adjuvants that induced biased immune responses, CDNs produce balanced memory Th1/Th2/Th17 and cytotoxic CD8+ T cell. Ebensen *et al.* first reported in 2007 that s.c. immunization of CDG/β-galactosidase (β-Gal) not only mounted a significant humoral response as compared to only β-Gal but also a cellular response in the spleen of immunized mice [Ebensen T, et.al., Clinical and Vaccine Immunology 2007;14(8),952-958]. The enzyme linked immunospot (ELISPOT) analysis of spleen cells showed that CDG immunization enhanced β-Gal specific memory Th1 and Th2 cells, thereby validating that CDG can modulate both humoral and a balanced Th response in a murine model. In 2010 and 2011, it has been shown that CDG and cyclic di-inosinemonophosphate (CDI) were potent inducers of interleukin (IL)-17a secreting CD4+ Th cells along with Th1 and Th2 responses when immunized intranasally.

CDNs have a potent anti-tumor activity. Demaria *et al.* showed that intratumoral injection of cGAMP enhanced the anti-tumor CD8+ T cell response inhibiting the growth of injected tumors in mice models of melanoma and colon cancer. Intratumoral injection of cGAMP in B16F10 lung metastasis induced a systemic CD8+ T cell response to restrict the growth of distant tumors. A study by Francica *et al.* showed that the CDN mediated tumor necrosis factor (TNF)-α production by innate immune cells are responsible for acute tumor clearance, and blocking TNF-α inhibits tumor necrosis and clearance against "cold" tumors [Francica BJ, et.al., Cancer Immunol. Res. 2018,6,422-433].

Lastly, CDNs effectively induce dendritic cell (DC) cross-presentation to the MHC class I molecules to produce a CTL response. A potent antigen-specific CD8+ memory T cell response by *in vivo* targeting of the DEC-205 receptor in DC, thereby provided an effective approach to target viral infections [Volckmar J, et.al., Vaccine 2019;37,4963-4974]. Combinatorial methods such as intramuscular immunization of CDA with alum have shown significant enhancement in antigen-specific CD4+ and CD8+ T cell responses while maintaining an excellent safety profile.

In 2007, Ebensen *et al.* first demonstrated CDNs as a mucosal immunoenhancer. They showed that CDG/β-Gal i.n. immunized mice not only mounted a systemic IgG response but also generated an enhanced β-Gal specific IgA response after 42 days in lung bronchial alveolar lavage fluids (BALF) and vaginal lavage. Intranasal immunization of CDG/β-Gal also enhanced the serum IgG2a and IgG1 production as compared to only β-Gal immunized mice. The systemic cellular response was also enhanced by CDG/β-Gal immunization, as was observed with the production of interferon (IFN)-γ, IL-5, and IL-2 in the *ex vivo* recall assay compared to only β-Gal immunized mice [Ebensen, 2007, see above].

CDNs also induce mucosal Th17. The mucosal adjuvant potential of CDNs was further strengthened when Madhun *et al.* showed that i.n. administration of a plant-based H5N1 influenza antigen induced high frequencies of multifunctional Th1 cells. Two doses of the CDG adjuvanted vaccine were able to mount a very high mucosal IgA response and protected against antigenically drifted H5N1 viruses. In comparison, the same vaccine when it immunized i.m. did not generate a Th1 response, which is critical for viral infections [Madhun AS, et.al., Vaccine 2011;29:4973-4982].

CDNs have been described as useful adjuvants in patent literature, for example the variants of CDA including thiophosphonate derivatives. Not only of c-di-AMP are described but also c-di-GMP or combinations of c-di-AGMP.

Although CDNs have shown promising and safe mucosal vaccine activities in animal models, at present there is no evidence on the capacity of these products to enhance the avidity of the antibody response in vaccine studies associated to the abrogation to the state of non-responsiveness. In fact, some results are distinct from the CDN-STING-type I IFNs-immunity paradigm, Andrew Mellor's group described a CDN-STING-type I IFNs-IDO1-tolerance signaling that mediates potent T-reg responses and suppresses inflammation. They first showed that DNA nanoparticles (DNP, containing PEI and plasmid DNA lacking TLR9 ligands) activate STING-dependent IDO-1 production that induces tolerance [Huang L, et.al., J. Immunol. 2013;191,3509-3513]. Later, they showed that the CDG treatment delayed the experimental autoimmune encephalitis (EAE) onset and reduced disease severity [Lemos, 2014]. They identified a new CDG-STING/type I IFNs/IDO1/Tregs pathway that suppresses central nervous system (CNS)-specific autoimmunity. Recently, the same group showed that the CDN-STING-IDO-1 activity in the tumor microenvironment (TME) promoted the growth of low antigenicity Lewis lung carcinoma (LLC). Lastly, they showed that treating pre-diabetic NOD mice with cGAMP delayed the type I diabetes (T1D) onset that depends on type I IFNs. Thus, CDN may be used as immune-modulatory drugs to abrogate the autoimmune responses as well as to induce strong Th1 immune responses due to their balanced profile of immune modulation. These evidences suggest that the effect and mechanisms of action of CDN significantly change according to the specific conditions, such as the vaccine antigen, the vaccine formulation and the vaccination scheme, thereby making extremely difficult to predict for a person skilled in the art.

### Detailed description of the invention

The present invention relates to the unmet needs in the field of vaccinology, specifically to the area of biological response modifiers (BRMs) able to abrogate the status of no responsiveness, administered alone or in combination with other innate immunity enhancers, aimed at controlling an infectious disease by the stimulation of the adaptive and innate immune stimulatory pathways. The subject of the present invention is the use of CDN as a biological response modifier to abrogate the non-responsiveness to particular pathogens/antigens by inducing a high avidity immune response capable of preventing the disease caused by infections. In particular, the present invention targets the viruses with the capacity to escape or evade the mechanisms of immune surveillance and consequently suppress the induction of a high avidity and effective adaptive response. This also applied to pathogens with increased genetic plasticity, which are able to evolve into variants with mutations in critical regions of their antigens preventing the immune clearance.

In a first aspect, the invention relates to CDNs for use as a BRM for improving the immune response of subjects in need thereof with underperforming immune systems, characterized by low/non-responsiveness to vaccine antigens or underperforming immune response to pathogens.

As used herein, the term "biological response modifier" refers to a compound with capacity as immunomodulatory agent; defined as the class of medications that target the disease-causing mechanism, which will directly impact in the mechanism related to a disease, for example, the absence or reduced maturation of affinity during the natural infection with coronavirus, or the absence of immune response to antigens as in the case of chronic diseases due to defects in the innate immunity mechanisms related with antigen presentation, costimulation, cytokine profile of immune responses. In such cases, the effect of the CDN will counteract the effect of the virus in the setting of prevention, post exposure prophylaxis or preventive therapy.

The term "underperforming immune system" refers to the immune system from individuals with immunodeficiency caused by the age (immunosenescence), a chronic condition, as for example the chronic diseases causing immunodepression and also includes the immune response of subjects with other chronic conditions like obesity, high blood pressure, diabetes that may represent a factor of risk for a severe condition or death causes by pathogens like (but not restricted to) coronaviruses. It also encompasses individuals with an underperforming immune system as a result of therapeutic interventions, such as pharmacological therapies (*e.g*. treatment with corticosteroids or immune suppressive drugs) or medical interventions. In the present set of examples, it is presented as a Balb/c mice with specific deficiencies in their innate immunity receptors.

As used herein, the term "low/non-responsiveness to vaccine antigens" refers to the subjects with an immune response considered as suboptimal for the protection against specific pathogens, while the term "underperforming immune response to pathogens" identify the subjects with an immune response considered as suboptimal to prevent the severity or death caused by a natural infection.

The composition according to the present invention being a compound according to the formula (I) 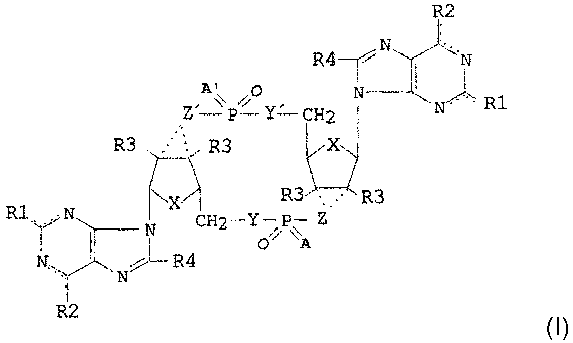 wherein
A, A' is independently from one another S or O;
X is independently from one another S, N, O, CH₂;
Y, Y' is independently from one another NH, CH₂, O;
Z, Z' is independently from one another NH, CH₂, O;
R₁ is independently from one another H, NH₂, S or O;
R₂ is independently from one another NH₂, O, H, or S;
R₃ is independently from one another absent if a covalent bond is present between the Z or
Z' and the C atom, or is hydrogen, OH, halogen, a straight or branched C₁-C₆ alkyl group, or a straight or branched C₁-C₆ alkoxy group which may optionally be substituted;
R₄ is independently from one another hydrogen, halogen, or a straight or branched C₁-C₆ alkyl group, which may optionally be substituted;
... is a single or double bond;
or conjugates thereof, and salts or solvates thereof.

In an embodiment, the biological response modifiers (BRMs), present in the immunogenic composition according to the present invention being a compound according to formula (I) wherein any one of the purine residues is an adenine, xanthine or hypoxanthine residue of combinations thereof. In an embodiment, both purine residues are adenine. That is, it is preferred that the cyclic di-nucleotide is a CDA. For example, the c-di-AMP is a 2,3'-c-di-AMP or 3,3'-c-di-AMP. Further, the BRMs may be a biphosphatethioate analogue of the c-di-AMP.

In an embodiment, the BRMs of general formula (I) is a compound wherein R3 is an OH group, X is an oxygen and Y, Y', Z and Z' are oxygen. Further, the compound according to formula (I) representing a BRM present in the immunogenic composition is anyone of the claims, characterized in that the compound according to formula I is a cyclic bis (3'-5') diadenylic acid (CDA or c-di-AMP), c-di-GMP, c-di-GAMP, c-di-IMP, c-IAMP, or a CDA thiophosphate, c-diMP Thiophosphate and cAMP-IMP thiophosphate.

According to an embodiment of the present invention, a CDN, like c-di-AMP, is used to enhance the avidity of the antibody response in a formulation comprising subunit antigens with a physical and chemical nature that result in non-responsiveness after multiple intranasal (IN) immunization, or resulting in low immunogenicity after administration to a subject deficient in antigen detection. Thus, the CDN, like c-di-AMP, is used particularly to overcome the non-response as well as to ensure the quality of the immune response by the inducing a strong, fast and high avidity antibody response in an otherwise low-/non-responsive subject. Such antibody responses are characterized by their enhanced functionality suppressing the binding of an infectious agent to their receptors, in particular a virus, and by the strong infectious agent/virus-specific neutralizing or killing activity.

The subject matter of the present invention also comprises the resulting formulations of c-di-AMP and said antigens as well as the potential combination of c-di-AMP with other immune stimulators to improve the effect of c-di-AMP, allowing dose splitting or adding complementary properties to the resulting formulation, in particular in terms of effector functions responsible for the clearance of infections, as well as enhancing the innate immunity activation with agonists of pattern recognition receptors, such as TLRs. The proposed invention also includes the method of preventing coronavirus infections, disease severity and transmission by administering the formulations subject of the present invention to a healthy person, to those at risk of infection or severe disease, or to a recently infected subject, preferably by the nasal or mucosal route, in a dose range capable of eliciting an effective antibody response, blocking the infection process, in particular at the entry site (*e.g*. nasopharyngeal mucosa), as well as reducing or preventing person to person horizontal transmission of the infectious pathogen, such a virus, of interest.

The present application is related to the use of CDN, like c-di-AMP, to induce a high avidity antibody response and consequently increase the anti-infective, in particular antiviral effect of the resulting antibody response. In a preferred embodiment, the formulations of the present invention are administered by the mucosal route, such as intranasal (IN), sublingual, intra-tracheal or pulmonary, to induce a strong antibody response at systemic and/or mucosal level, such as in blood and/or mucosal secretions or tissues. The present inventors recognized that the antibody response was superior in strength, compared to the administration of the same antigen dose in aluminum hydroxide, as a control vaccine. The formulations including the CDN as well as the specific use of CDN according to the present invention induce a mucosal IgA response with ACE2-binding inhibitory capacity and high levels of viral neutralizing titers. The SARS-CoV-2 antibody response, the ACE2-RBD binding inhibitory effect and the viral neutralizing response are dependent on the use of c-di-AMP by the IN route of administration.

One specific embodiment of the present invention comprises the formulations of c-di-AMP and manosylated subunit antigens produced, and more specifically the RBD proteins and other proteins from SARS-CoV-2 or other infective agents, in particular coronaviruses, as well as the method of inducing an immune response in mucosal and systemic compartments with the use of these formulations by IN administration. These formulations are able to abrogate the low-or non-responsiveness status in the specific host with deficiencies in the expression of receptors, those needed to process the antigens, resembling the process of lower innate immunity receptors that occurs in elderly because of immune senescence or the affected immune receptors in immune-depressed persons. In the specific case of the examples, the Balb/c mice have specific deficiencies in the expression of innate immunity receptors by their professional antigen presenting cells (APCs) implying a non-response to the antigen by the IN route and the weak response after parenteral administration. This effect can in turn be translated to the framework of human subjects who are poor responders to vaccination in the clinic (as described in the following). As demonstrated in the examples, the subject matter of the present invention are able to induce a mucosal IgA response with ACE2-binding inhibitory capacity as well as a superior ACE2-binding inhibitory effect in the sera compared to the response elicited by the same dose of the antigen administered by the intramuscular (IM) route in aluminum hydroxide. The formulations of the present invention induce a stronger viral neutralizing response as compared to the same antigen, administered by the IN route without c-di-AMP or formulated with the TLR agonists in the setting of non-responsiveness due to the lower expression of receptors in professional antigen presenting cells. The coadministration in the same formulation of a TLR agonist, preferably the hepatitis B virus core antigen (HBcAg), constitute another embodiment provided that the subject requires additional stimulation of the innate immune response, as this is the case of immunodepressions caused by disease or immune senescence.

According to the state of the art, it was expected that a compound with demonstrated TLR3, TLR7 and TLR8 multi-agonist effect, such as the HBcAg and previously used as adjuvant, may also enhance the immune response to the RBD produced in *Pichia pastoris*; however, mice with low levels of mannose receptors and consequently non-responsive to this glycosylated antigen (heavily manosylated Pichia derived antigen RBD, being Pichia a cell factory broadly used for vaccine antigen production) was refractory to the induction of an immune response in absence of c-di-AMP. The lack of immune response of the formulations comprising HBcAg and RBD and the high immunogenicity of the CDA comprising formulation evidenced the capacity of c-di-AMP to abrogate the status of non-responsiveness, completely unexpected and surprising in their potency and functional activity in front of the absence of effect of the recognized adjuvant HBcAg. The non-responsiveness of the Balb/c mice strain resulting from the low level or absent mannose and other innate immunity receptors in their APCs resembles the low level of expression of innate immunity receptors in the elderly and other immune depressed subjects, naturally low responders to vaccines when compared to the younger population.

Older adults and people with underlying health conditions are at higher risk of developing severe clinical manifestations of COVID-19. This could be associated with less responsive innate immunity ligands, altered immune responses-both in quality and quantity-changes in the cytokine milieu and lower expression of co-stimulatory signals. Thus, intrinsic host factors, like the reduction of the innate immune system to sense the PAMPs, may contribute to disease severity and mortality.

No antibody response was detected in Balb/c mice by nasal immunization in the groups immunized with RBD in HBcAg in the two doses of RBD studied. Surprisingly, c-di AMP abrogated the state of low/non-responsiveness against the manosylated antigen by IN route, in addition, it also induced a superior response in terms of avidity compared to the alum formulation administered by IM route, the two activities have no precedent in the scientific literature related to the field of CDN and the potentiation of the avidity is a rare characteristic/property of unique adjuvant formulations. Thus, there are two relevant aspects in connection with the use of c-di-AMP. First, the capacity of this compound to abrogate the state of non-responsiveness in the experimental setting using the well-established preclinical mouse model, with low expression levels of mannose and other innate immunity receptors. Second, the capacity of the resulting formulation to induce an antibody response with remarkable avidity, strength and functionality, alone or in combination with HBcAg.

A relevant characteristic of the formulation, subject matter of the present invention, refers to the use of CDN, like c-di-AMP to generate an immune response with a rapid dynamic of increase in titers, which represent a desired capacity of the immune response triggered by a vaccine, in particular in the context of a pandemic/epidemic outbreak and travelers, the capacity to protect the majority of the vaccinated subjects in a short period of time and the lower number of required doses.

In an embodiment, the pathogen or infectious agents are organisms that are capable of producing infection or infectious disease. They include bacteria, fungi, viruses, and parasites. In an embodiment, the pathogen is a virus and the vaccine is against said virus. The term "virus" includes the embodiments of coronavirus, influenza virus, respiratory syncytial virus, rhinovirus, including DNA virus and RNA virus. In an embodiment, the virus is a virus of the dengue virus four serotypes.

In a preferred embodiment of the present application, RBD or S antigens of the SARS-CoV-2 from different variants or other coronavirus or infectious agent, one or more, are included in the formulation with c-di-AMP or CDN in general under the appropriate conditions of concentration.

In another embodiment of the present application, the formulation of RBD and c-di-AMP or CDN in general is combined with other antigens of the same virus (SARS-CoV-2) or from the same family of virus (beta-coronavirus) in order to increase the valency of the formulation and to extend the properties of the formulation to the rest of the antigens.

In another embodiment of the proposed application, CDN, like c-di-AMP, is formulated with antigens from coronavirus, influenza virus, respiratory syncytial virus, rhinovirus and any other relevant respiratory pathogen with immune escape/evasion capacity or when stimulation of high affinity antibodies is critical for clearance, combined in a multivalent vaccine formulation aimed at preventing acute respiratory infections in general. In another embodiment of the proposed application, CDN, like c-di-AMP, is co-administered with adjuvants favoring the depot effect and slow release of the BRMs and the antigens with the aim of reducing or splitting the dose of the antigen in the vaccine composition. The co-administered adjuvants are (not restricted to) the following: alum, PLGA, chitosan, ISCOMs, ISCOM-matrix and biodegradable micro- or nanoparticles.
Moreover, the CDN are useful to improve an immune response by boosting for accelerating the germinal center reaction to further foster an antibody hypersomatic mutation and affinity maturation. In this connection, the term "accelerating the germinal center reaction" refers to the situation where the antibody repertoire of the B-cells mature by mutation known as hypersomatic mutation and affinity maturation. This allows production of antibody producing B-cells eventually being antibody producing plasma cells (effector B-cells) as well as memory B-cells with higher affinity antibodies.

In another embodiment of the present application the formulation is administered by mucosal and/or parenteral routes, being the preferred routes the IN route and the IM route. The formulation is intended for mucosal, in particular the IN, route; however, it can be applied by mucosal and parenteral routes simultaneously, as well as sequentially in a prime-boost or prime-pull scheme. Similarly, can be used as a priming or booster of mRNA, DNA, non-replicative or replicative live vectors or vaccines based in recombinant proteins for SARS-CoV-2, as well as other coronaviruses, in order to promote in the immunized patients a partial increase of the properties described for the present formulation.

As used herein, the term booster refers to the treatment/vaccination of subjects that has been previously treated with another vaccine composition. Prime-boost refers to the rational design of an immunization protocol to induce active immunity based in the administration of two different vaccines, where one of the vaccines is one of the vaccines described in the present invention. In addition, prime-pull protocols aimed at pulling cells into the mucosal territory of vaccination, thereby triggering also local/mucosal immune responses able to confer protection against not only disease but also infection, reducing the risk of horizontal transmission to a susceptible host.

In a preferred embodiment, the formulations will comprise the CDN, like c-di-AMP, in a range of doses allowing a stronger immune response according to the current pharmacological studies, the range comprise the doses from 0.01 mg up to 0.4 mg of CDN in phosphate buffered saline solution (PBS) per immunized subject, a similar range of dose of HBcAg may be coadministered, further enhancing the BRM effect of CDN in the formulation of both compounds as well as in their combination with several antigens in mono- and multivalent formulations.

In a preferred embodiment, the formulations of the present invention can be administered by parenteral route as part of a formulation with alum or other depot adjuvants, combined with non-adjuvanted vaccines, formulated with different adjuvants or just mixed at the moment of product administration with a different vaccine for SARS-CoV-2, coronavirus, or against a different infectious agent(s). In addition, the BRMs or the mixture of BRMs of the present invention can be provided alone without the administration of antigens.

In a preferred embodiment of the present application, the formulation will be administered in accelerated schedules of immunization considering the application every week, every 15 days or appropriately adjusted to the requirement, as well as in long-term schedules tailored to time the boosting with the contraction phase of the germinal center formation to further foster hypersomatic mutation and affinity maturation. The formulation can be applied to persons already exposed to SARS-CoV-2 or other coronavirus or respiratory infections.

The CDN for use according to the present invention may be in form of vaccine formulations comprising one or multiple antigens such as Spikes or RBD, as well as other vaccine-relevant proteins either purified from the pathogen itself or produced as a recombinant protein in a cell factory like *Pichia pastoris, Escherichia coli* or an eukaryotic cell based protein expression system, in particular from the group of recombinant SARS, IAV, VSR, dengue virus, HBV (HBsAg), HIV, HCV, Zika and other pathogens.

### Brief Description of figures

Fig. 1. Anti-RBD response induced after three immunizations, following the immunisation schedule described in example 1. Titres were assessed using an RBD protein obtained in mammal cells to avoid the response against the homologous glycosylation.
Fig. 2. Avidity of the anti-RBD response induced after three administrations, following the immunization schedule described in example 1. The CDA (G6) or the CDA-HBcAg combined formulations (G10 and G11) significantly stimulated the avidity of the antibody response against RBD. The avidity index is defined by the percent of the antibody bound to the RBD in respect to the untreated control, according to the molar concentration of Guanidium chloride (GuHCl, the chaotropic agent). The concentration of GuHCl that reduces in 50% the optical density by ELISA.
Fig. 3. Inhibitory activity of the antibody response induced by the groups with the strongest anti-RBD response after three administrations, following the immunization schedule described in example 1. A) G2; B) G4; C) G6; D) G11; E) G12 and F) Monoclonal antibody (McAb) CBSSRBD-S.8 used as standard curve to plot the results in the five ELISA plates.
Fig. 4. SARS-CoV-2 neutralizing activity of the antibody response induced by the groups with the strongest anti-RBD response after three administrations, following the immunisation schedule described in example.
Fig. 5. Anti-RBD IgA response induced in neutralizing activity of the antibody response induced by the groups with the strongest anti-RBD response after three administrations, following the immunization schedule described in example.
Fig. 6. Anti-RBD response induced after three immunizations, following the immunisation schedule described in example 13. Titers were assessed using an RBD protein obtained in mammal cells to avoid the response against the homologous glycosylation.
Fig. 7. Kinetic of the anti-RBD IgG response up to day 150.
Fig. 8. Inhibitory activity of the antibody response induced by the groups with the strongest anti-RBD response after three administrations, following the immunization schedule described in example 13.
Fig. 9. Secretion of IFN gamma (A) and IL5 (B), detected in supernatants of 96 h cultured splenocytes from selected groups of mice immunized in Example 1. Cells were stimulated with the homologous (Pichia pastoris produced RBD), heterologous (CHO produced RBD), Concanavalin A (ConA) and PBS during 96 h and supernatants were studied using Luminex assay.
Fig. 10. Viral neutralization studies using neutralization assay specific for the original Wuhan variant, the Delta variant and Omicron (BA4/BA5 subvariant). Serum samples from Example 1 immunization schedule were individually tested.

### Examples

### Example 1: The use of CDA abrogates the non-responsive nature of manosylated RBD in Balb/c mice and induces a fast and strong immune response.

Several vaccine formulations were studied to select the composition with the most attractive immune response for SARS-CoV2 prevention. The receptor-binding domain (RBD) protein was used in combination with other compounds with immunostimulatory activity. The protein RBD was obtained as a recombinant glycoprotein in *Pichia pastoris,* with the characteristic manosylated pattern of glycosylation.

To explore the effect on RBD nasal immunogenicity, CDA, produced by enzymatic synthesis, was used alone or in combination with the Hepatitis B core antigen (HBcAg), as an innate immune stimulating formulation, by a simple combination with RBD.

The formulations tested in the present study are described in table 1. Balb/c mice deficient in the expression of innate immunity receptors were used, specifically females, 8 per group, 12 groups, 96 mice in total, received 3 administrations on days 0, 14 and 28 by the IN (G1 - G11) and IM (G12) routes. The doses of RBD used in the study were 12.5 µg and 50 µg per mouse. The doses of CDA were 5 µg and 10 µg per mouse and the dose of HBcAg used was 10 µg / mouse. Serum samples were taken on days -3, 25 and 42.

**Table 1. Description of the formulations and schedules of immunisation, Example 1.**

| **Group** | **Formulations (concentration of the components in PBS)** | **Dose (µg/mice)** | **Total Vol. (Vol./nostril)** | **Route** | **N** |
|---|---|---|---|---|---|
| 1 | PBS (Placebo) | - | 50µL (25µL) | IN | 8 |
| 2 | CDA: 0.1 mg/mL | CDA: 5µg | 50µL (25µL) | IN | 8 |
| 3 | RBD: 0.25 mg/mL | RBD: 12.5µg | 50µL (25µL) | IN | 8 |
| 4 | RBD: 1.0 mg/mL | RBD: 50µg | 50µL (25µL) | IN | 8 |
| 5 | RBD: 0.25 mg/mL + CDA: 0.1 mg/mL | RBD: 12.5µg | 50µL (25µL) | IN | 8 |
| | | CDA: 5µg | | | |
| 6 | RBD: 1.0 mg/mL + CDA: 0.1 mg/mL | RBD: 50µg | 50µL (25µL) | IN | 8 |
| | | CDA: 5µg | | | |
| 7 | RBD: 1.0 mg/mL + CDA: 0.2 mg/mL | RBD: 50µg | 50µL (25µL) | IN | 8 |
| | | CDA: 10µg | | | |
| 8 | RBD: 0.25 mg/mL + HBcAg: 0.2 mg/mL | RBD: 12.5µg | 50µL (25µL) | IN | 8 |
| | | HBcAg: 10µg | | | |
| 9 | RBD: 1.0 mg/mL + HBcAg: 0.2 mg/mL | RBD: 50µg | 50µL (25µL) | IN | 8 |
| | | HBcAg: 10µg | | | |
| 10 | RBD: 0.25 mg/mL + CDA 0.1 mg/mL+ HBcAg: 0.2 mg/mL | RBD: 12.5µg | 50µL (25µL) | IN | 8 |
| | | CDA: 5µg | | | |
| | | HBcAg: 10µg | | | |
| 11 | RBD: 1.0 mg/mL + CDA 0.1 mg/mL+ HBcAg: 0.2 mg/mL | RBD: 50µg | 50µL (25µL) | IN | 8 |
| | | CDA: 5µg | | | |
| | | HBcAg: 10µg | | | |
| 12 | RBD 0.5 mg/mL + 0.5mg/mL Alum | RBD: 50µg | 100µL | IM | 8 |
| | | Alum: 50µg | | | |

| | | | | | |
|---|---|---|---|---|---|
| CDA: c-di-AMP | | | | | |

On day 25th, only the mice immunized with c-di-AMP induced a strong Ab response (see table 2). The responses induced with the formulations tested in groups 5, 6, 10 and 11 were superior to the immune response induced in the groups with the antigen administered alone and even in the groups were the RBD was administered with HBcAg alone (groups 8 and 9). The intensity of the response by the IN route was remarkable considering that the antibody response was tested as soon as on day 25^{th}, 11 days after the second dose. The intensity was even stronger as compared to the group immunized by IM route (G12). Interestingly, the higher (double) dose of CDA tested in group 7 resulted in a lower immune response after the second dose, demonstrating that the effect of CDA can be enclosed in a certain range of doses and that not necessarily the higher doses represents the most effective scenario to abrogate the immune response.

**Table 2. Individual anti-RBD IgG levels in serum samples of individual mice from the 12 groups of the study.**

| G1 | G2 | G3 | G4 | G5 | G6 | G7 | G8 | G9 | G10 | G11 | G12 |
|---|---|---|---|---|---|---|---|---|---|---|---|
| neg | neg | neg | neg | 5358 | 12298 | neg | neg | neg | 545 | 678 | 193 |
| neg | neg | neg | neg | 742 | 12635 | neg | neg | neg | 6813 | 2920 | 512 |
| neg | neg | neg | neg | 4378 | 3868 | 324 | neg | neg | Neg | 2598 | 489 |
| neg | neg | neg | neg | 3537 | 9199 | 211 | neg | neg | 3173 | 368 | 454 |
| neg | neg | neg | neg | 895 | 11996 | 302 | neg | neg | 5345 | 5544 | neg |
| neg | neg | neg | neg | 87 | 11802 | 218 | neg | neg | 609 | 5064 | 238 |
| neg | neg | neg | neg | 80 | 9589 | 516 | neg | neg | 6135 | 4010 | 218 |
| neg | neg | neg | neg | 185 | 5776 | neg | neg | neg | 3894 | 352 | 252 |

The protein RBD, obtained as a recombinant glycoprotein in *Pichia pastoris,* with the characteristic manosylated pattern of glycosylation, resulted in a non-detectable immune response when administered in Balb/c mice. This effect is the result of the characteristic absence of mannose receptors in Balb/c dendritic cells [Autenrieth SE, Autenrieth IB. Immunology 2009;127(4):523-529].

Surprisingly, Balb/c mice with deficiencies in their innate immunity receptors, immunized with manosylated RBD, resulted a valuable model to demonstrate that the non-responsiveness due to lower expression or absence of innate immune receptors can be abrogated with the use of CDA. Only the five groups using CDA were able to induce an immune response. Even a previously used adjuvant for nasal formulations (HBcAg) was unable to induce an immune response when HBcAg was administered with RBD in absence of CDA.

In the case of persons with immune-deficiencies or immune senescence, the level of expression of innate immunity receptors is depressed. The surprising effect of abrogation of the non-responsive status and the induction of a strong immune response detected in the present study open a novel opportunity to create vaccines for persons with a reduced level of immune responsiveness due to lower expression of innate immune receptors, as this is the case of immune depressed or immune senescent persons [Metcalf TU, et.al., Aging Cell. 2015 Jun;14(3):421-32. doi: 10.1111/acel.12320].

### Example 2: The combination of CDA with HBcAg favours the split of the immunizing dose.

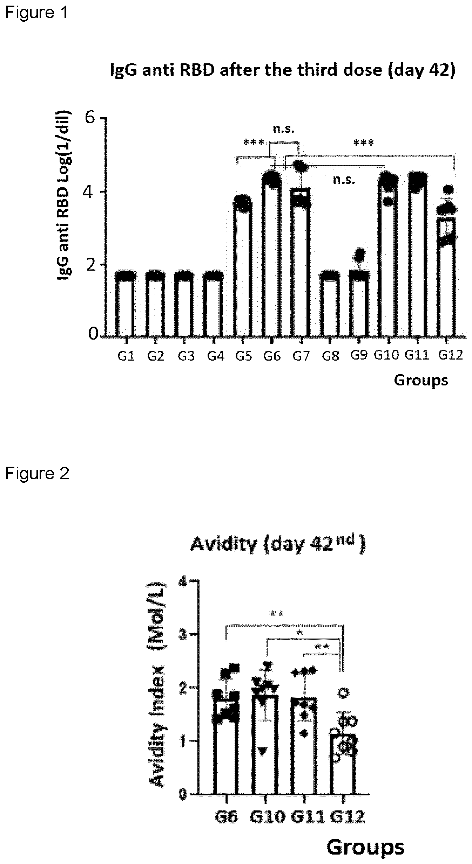

Further studying the effect of the different formulations after three immunisations, it was re-confirmed the absence of response in the groups immunized without CDA by IN route (G3 and G4). Even in the groups immunized with the HBcAg, a generalized absence of response was detected (G8 and G9), with the exception of two mice that developed a slight antibody response (Fig. 1). Considering the absence of effect of HBcAg found on G8 and G9 when this immunostimulating compound is administered with RBD alone, it was also surprising to detect that the group with the lower dose of RBD and both compounds (CDA and HBcAg, G10) induced, after three doses, an immune response similar in intensity compared to the groups with the higher dose of RBD (G6 and G11, p>0.05), demonstrating that the dose of RBD may be split four times, without compromising the effect detected in the groups with the higher doses. This effect was not detected between the group with the RBD and CDA alone (G5), that continues to induce a significantly inferior Ab titres against RBD compared to G6 (p<0.001). These results demonstrate that only after the abrogation of the non-responsiveness, the HBcAg effect was detectable (see fig. 1).

### Example 3. The anti-RBD antibody response induced by CDA-comprising compositions is characterized by strong avidity.

Studying the avidity of the antibody response elicited by the different formulations described in example 1, after three immunisations, it was detected a significantly superior avidity in the serum samples from groups immunized with CDA or the combined formulation of CDA and HBcAg, compared to the group immunized with the same protein, at the same dose, using the IM route of immunisation.

The development of novel compounds with the capacity of inducing high avidity antibodies represents a challenge in vaccinology. Different adjuvants enhance the immune response producing a strong antibody response, or Th1 cellular response; however, the substances with the capacity to induce a high avidity antibody response are limited. New formulations have been developed to solve this unmet need of current vaccines. The formulation may have different properties compared to the use of the TLR agonists alone and indicate that the combined composition is a promising formulation to solve this unmet need of current vaccines (Lu, 2019). This is the first time that CDA alone or in combination with HBcAg demonstrates an immunoenhancing effect on the avidity of the immune response. This characteristic of the resulting immune response is essential in particular against viruses with the capacity to escape or evade the mechanisms of immune surveillance and consequently suppress the induction of a high avidity and effective adaptive response, evidencing the industrial applicability of the novel subject matter of the present invention, see Figure 2.

### Example 4. Functionality of the resulting immune response: inhibitory effect of the induced antibodies on antigen-receptor interaction.

A cyclic di nucleotide (CDN) like c-di-AMP demonstrated a surprising capacity to abrogate the non-responsiveness and to enhance the avidity of the antibody response in a formulation comprising subunit antigens with a physical and chemical nature that result in non-responsiveness after multiple intranasal (IN) immunization. Thus, the c-di-AMP was able to overcome the non-response in the studied setting, also inducing a strong, fast and high avidity antibody response in the otherwise low-/non-responsive subject. Thus, we studied the antibody response effect on the ACE2-binding inhibitory activity.

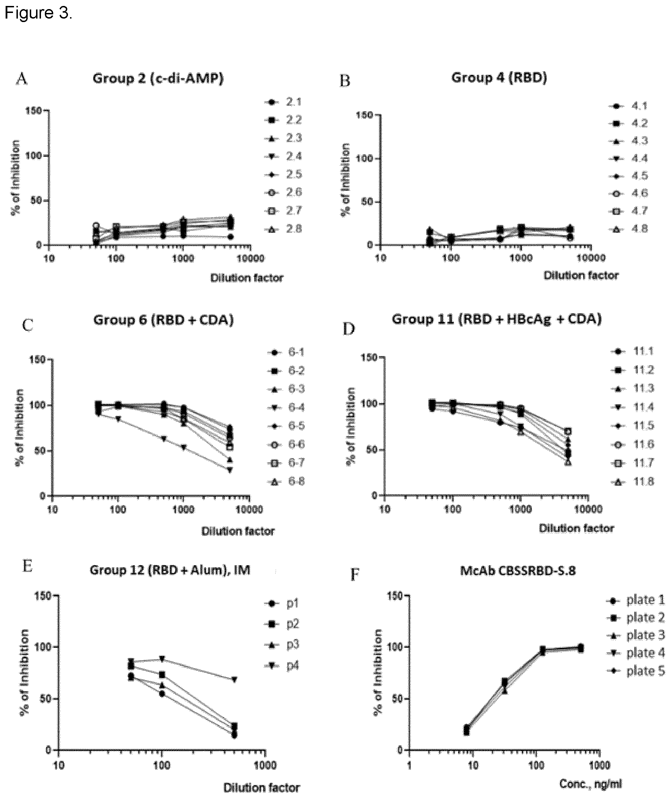

The resulting antibody response is capable of inhibiting the binding of the antigen to their cellular receptor ACE2, a process that resembles the interaction of the virus to their cellular receptor. This inhibitory effect is essential to ensure the strong antiviral effect of the immune response against an infectious agent/virus through the specific neutralizing activity. It is demonstrated a strong inhibitory effect induced by the CDA or the CDA combined to HBcAg (Fig 3C and D) when compared to the group immunized with RBD alone (Fig 3B). This functional aspect of the antibody response is linked to the increased quality of the same response in terms of avidity. The antibody response induced in G12, the group immunized by the IM route, was remarkably inferior in terms of inhibitory effect on RBD-ACE2 recognition. Although G12 results were shown in four pools of serum samples due to reduced sample availability, it was clearly demonstrated the significantly reduced ACE2-binding inhibitory effect compared to the groups with the presence of CDA or CDA/HBcAg combined formulations (Fig 3E). The quality of the experiment was ensured with the use of a McAb in the standard curve of the five plates used in the study and the reproducibility of the inhibitory effect (Fig 3F) as well as with the assessment of the ACE2 inhibitory binding in a group that received the placebo of CDA but no antigen (Fig 3A).

### Example 5. Functionality of the resulting immune response: SARS-CoV-2 neutralizing activity of the induced antibodies.

In example 1, the c-di-AMP (CDA) efficiently abrogated the non-responsiveness against RBD and subsequently, it was shown the enhanced the avidity of the antibody response induced with a formulation comprising an antigens with the physical and chemical nature resulting in non-responsiveness after multiple intranasal (IN) immunization when used in absence of this BRM.

The c-di-AMP demonstrated a surprising capacity to overcome the non-response as well as to ensure the quality of the immune response by inducing a strong, fast and high avidity antibody response by the nasal route of immunization that was able to inhibit the ACE2-binding capacity of the RBD recombinant protein. In the present example, we studied the effect of the formulations described in example 1, in the resulting SARS-CoV-2 virus neutralizing activity.

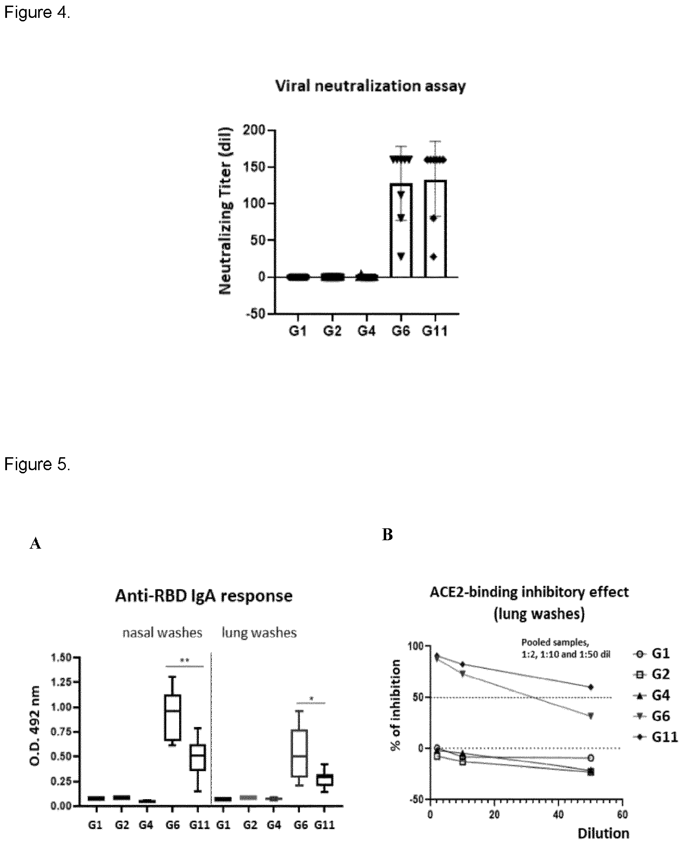

Viral neutralization assay was conducted in groups 1, 2, 4, 6 and 11. The samples were individually assayed. Viral neutralization responses (viral neutralization vs Wuhan isolate) was only detected in groups: G6 (RBD + CDA) and G11 (RBD + CDA + HBcAg). In both groups, almost all samples evidenced saturated levels at 1:160 dilution (see fig. 4).

A non-detectable response was obtained at 1:5 dilution in the groups: G1 (PBS); G2 (CDA Placebo) and G4 (RBD 50ug IN without CDA), (all mice were negative at 1:5 dilution). SARS-CoV-2 neutralization response was consistent with the ACE2 inhibitory activity demonstrated in Example 4.

The CDA BRM was able to induce a strong anti-RBD response when mixed with the protein produced in Pichia pastoris and this response evidenced the functionality of the Ab response by inducing a strong neutralization of the virus against the homologous strain Wuhan isolate). Group 4 was unable to generate Ab response, even at the RBD dose 50ug/mouse and had a similar behavior compared to the Placebo (G2) and negative control (G1).

These results further support that Balb/c mice are non-responders to Pichia RBD via IN route in absence of CDA and the novelty of the surprising findings that demonstrate that, with the use of CDA or their formulation with HBcAg, the antibody response a) is induced; b) is strong, c) has stronger avidity compared to the traditionally induced response by IM route in alum, with a remarkable functional activity in terms of ACE2 inhibitory activity as well as virus neutralizing capacity.

### Example 6. Functionality of the resulting immune response: SARS-CoV-2 neutralizing activity on several SARS-CoV-2 variants.

The c-di-AMP demonstrated a surprising capacity to overcome the non-response as well as to ensure the quality of the immune response by inducing a strong, fast and high avidity antibody response by the nasal route of immunization that was able to inhibit the ACE2-binding capacity of the RBD recombinant protein and neutralize the SARS-CoV-2 virus *in vitro.* In the present example, we studied the effect of the formulations described in example 1, in the neutralization of different viral strains of SARS-CoV-2 virus. With this aim, different virus variants were assayed. The Wuhan original strain, the alpha, delta and omicron variants were studied. The study conducted in vitro evidenced the neutralizing effect against the different variants of serum samples from G6 and G11. No neutralizing activity was detected in serum samples of mice immunized without CDA.

### Example 7. On the immune response induced at mucosal surfaces of the respiratory tract.

To further characterize the immune response induced by the formulations described in Example 1, nasal and lung washes were collected from mice treated with three doses of RBD or placebo controls following the schedule and formulations described for G1, G2, G4, G6 and G11.

Following the same pattern of response, only the groups immunized with the RBD formulated with CDA (G6) or the combined formulation with CDA/HBcAg/RBD were able to induce a positive anti-RBD immune response, both in nasal and lung washes (Fig. 5A). The intensity of the IgA response was superior in G6, although the ACE2-binding inhibitory effect was higher in G11 (Fig. 5B). However, the apparent difference may be explained by detectable levels of IgG in the lung washes (data not shown).

The present results is consistent with the rest of the results as well as with the stat of the art that considers that, for achieving the control of a pathogen infecting mucosa, the most suitable scenario is to develop an immune response capable of protecting both at mucosal and systemic compartments, as the injectable products are not suitable to prevent person-to-person transmission. The mechanism explaining the surprising effect of non-responsiveness abrogation, a novel effect induced by the CDA, as well as the resulting impact of CDA or the formulation CDA-HBcAg on the intensity, quality and functionality of the immune response deserve further study and understanding.

### Example 8. Formulations for nasal administration.

Several dose-escalating studies have been designed and conducted in order to find the optimal conditions in terms of CDA dose using RBD with the sequence of Wuhan and Omicron isolates. The results reconfirmed the findings of a specific range of concentration for the CDA component. The c-di-AMP performs in an optimal range of concentrations allowing a stronger immune response from 0.1 mg/mL up to 0.2 mg/mL of c-di-AMP, both in single or combined (CDA/HBcAg) formulations for both variants of the same antigen.

In subsequent studies, it was demonstrated that the formulations of the present study could be applied by nasal and parenteral route. In the case of parenteral route, preferably by IM route as part of a formulation with alum and other formulations, as well as mixed at the moment of product administration. Similar results were confirmed using the S protein from different coronavirus, even using combination of RBD and S and other antigens from the same virus and in combined vaccines as part of a multivalent vaccine for several respiratory infections.

The formulations of the present invention have been administered in accelerated schedules of immunization: weekly or every 2 weeks, demonstrating a strong immune response induced in serum and mucosal secretions. The use of the CDA or CDA/HBcAg has been satisfactorily tested as a boost of already existing vaccines. The formulations has been satisfactorily applied to persons already exposed to SARS-CoV-2 or during the early stages of infection as part of a post-exposure prophylaxis treatment.

### Example 9. Study of different routes of immunization.

To characterize the immune response induced by the formulations 6 and 11 described in Example 1, groups of 8 mice were administered by intranasal, sublingual, intra-tracheal, pulmonary, intramuscular, subcutaneous, intradermal, transcutaneous and perifollicular routes. As control groups, mice received the excipient or the excipients, the RBD and the HBcAg at the dose and concentrations used in groups 6 or 11, in absence of CDN. Only the groups that received CDN and RBD developed an RBD specific immune response, the immunogenicity order according to the mucosal routes was: 1) IN, 2) pulmonary, 3) intra-tracheal. In the case of parenteral routes, 1) IM, 2) subcutaneous, 3) intradermal, 4) transcutaneous and 5) perifollicular.

### Example 10. The effect of different CDNs on the resulting biological response

To characterize the effect of the CDN nature on the resulting biological response, the formulations 6 and 11 described in Example 1, groups of 8 mice were administered by IN route with or without the RBD antigen, and each group comprising a different CDN.

The analysis of the immune response revealed that all CDN may behave as biological response modifiers capable of subverting the non-responsiveness against RBD and to promote antibody responses with strong avidity, inhibitory activity and viral neutralizing effect. However, some differences appeared demonstrating that the cyclic bis (3'-5') diodenylic acid (CDA) or c-di-AMP induced the superior responses in terms of antibody response and the avidity/inhibition and neutralization against SARS-CoV-2, the rest of the CDN under study induced a lower response in the following order of intensity: 2) c-di-IMP, 3) c-IAMP, or a 4) CDA thiophosphate, 5) c-di-IMP thiophosphate and 6) c-IAMP thiophosphate.

### Example 11. The use of the BRM on the pre/post exposure prophylaxis of infectious diseases

SARS, influenza A virus (IAV), syncytial respiratory virus, rhinovirus and dengue viruses are pathogens causing acute respiratory and/or febrile infections that has been adapted to produce infections in animal models. The use of the formulations exclusively comprising the biological response modifier (CDA) or its combination with HBcAg has produced a quantitative reduction of the viral titers of SARS and IAV in the models of acutely infected subjects and *in vitro* infected & treated cells. The *in vivo* treatment with the BRMs (CDA, or CDA/HBcAg) induced a high avidity antibody response against the viral antigens and the induction of antibody titers with capacity to neutralize these viruses. The effect was detected when the CDA or the combination CDA / HBcAg was administered before or after the infection, evidencing the prophylactic effect pre- and post- exposure. Similar results were found in the setting of chronic infectious diseases associated non-responsiveness where the viruses and major antigens of the HBV and HIV are reduced in studies conducted *in vitro,* increasing the scope of the present invention to the field of acute respiratory or febrile infections as well as the field of chronic infectious diseases. The *in vivo* concentrations of CDA or their combination CDA/HBcAg are similar to the optimal concentrations obtained with the RBD model antigen, both in the setting of vaccine formulations as well as in the setting of post exposure/early therapy where the CDA or the combination of CDA/HBcAg could be used without the presence of the antigen.

### Example 12. Self-administration of CDN-comprising formulations

The formulations comprising CDN and RBD was administered by a trained person or self-administered in a prospective, controlled and randomized clinical trial. The effect on the resulting immune response was studied in serum samples extracted after 1 booster in persons immunized by IM immunization and also in a schedule of three administrations of the IN formulation two or four weeks apart. All groups were studied partially immunizing the subjects using specialized nurses or alternatively using the same procedure as a self-administered product. No differences were found between the subjects immunized and self-immunized, suggesting that the use of BRM comprising compositions can be self-administered. The self-administration is an attractive feature for these compositions when administered by IN route.

### Example 13. Dose escalation of CDA in formulations with HBcAg/RBD or RBD

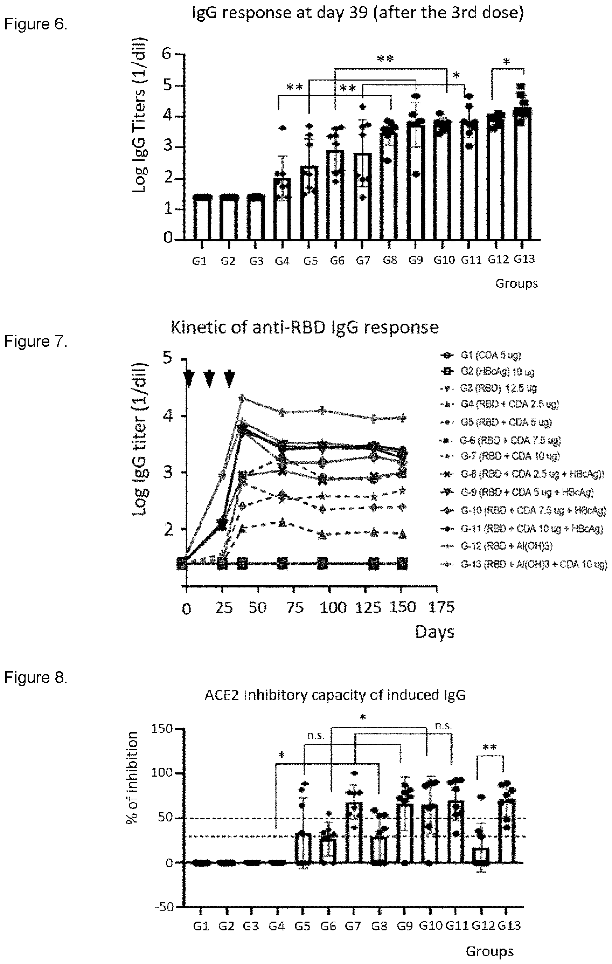

Thirteen groups of female Balb/c mice (6 per group in groups G1 to G3 and 8 per group from G4 to G13) were immunized on days 0, 14 and 28 by the IN (G1 to G11) and IM (G12 and G13) routes. The dose and concentration of RBD, Alum, CDA and HBcAg is presented in the Table 3 including the studied formulations. The assessment of the immune response was conducted after bleeding the mice on days -3 (preimmune) and after the second (day 25) and third doses (days 39, 67, 95,131 and 151). In the absence of CDA (G3, Figs 6 to 9), anti-RBD antibodies were not induced, therefore the effect found in previous studies is reproduced. The application of CDA generate detectable immune responses in CDA-RBD or CDA-RBD-HBcAg formulations, reversing the non-responsive state in the recipient organism through the IN administration route. The intensity of the anti-RBD IgG antibody response is dependent on the CDA dose and increases significantly with co-administration of HBcAg (G8 to G11, respectively). RBD formulations containing CDA (G4 to G7), CDA + HBcAg (G8 to G11) and Al(OH)3 (G12) and AL(OH)3 + CDA (G13) induced specific anti-RBD antibodies, which remain in the circulation for several months in a follow-up study after the 3rd immunization (Fig. 7).

The co-administration of CDA + Al(OH)3 and RBD as a combined formulation, by the IM route, increased the intensity of the anti-RBD response, compared to the formulation RBD + Al(OH)3 (without CDA). The finding of immune responses by the IM route confirmed that the absence of response (non-responsiveness status) is dependent on the immunization route, suggesting that the antigen uptake via IN route is dependent of different mechanisms and the non-responsiveness is dependent on the immunization route although the responses is enhanced with the addition of CDA, and in consequence the functionality of the Ab response has a dramatic increase as shown by the enhancement of the inhibitory function of the induced antibodies (G12 vs G13). It was surprising to find that similar or superior levels of IgG anti RBD induced by IN route induced a stronger inhibitory ACE2 response compared to the group 12, immunized with alum hydroxide by the IM route, evidencing the superiority of the formulation in terms of immune response functionality, and it may be due to the enhancement in the avidity of the resulting Ab response as shown in the present application as a similar Ab response is unable to induce a significant ACE2 inhibitory effect when compared to nasally induced groups (G7-G11 vs G12). The proportion of mice with inhibitory Ab response above 30% in nasally administered groups with CDA was significantly higher compared to the G12 (Figure 8).

**Table 3. Description of the formulations and schedules of immunisation, Example 13.**

| Group | Formulations (Concentration of the components dissolved in PBS) | Dose (µg/mice) | Vol./mice (µL) | Route | N |
|---|---|---|---|---|---|
| 1 | CDA: 0.1 mg/mL | CDA: 5 | 50 | IN | 6 |
| 2 | HBcAg: 0.2 mg/mL | HBcAg: 10 | 50 | IN | 6 |
| 3 | RBD: 0.25 mg/mL | RBD: 12.5 | 50 | IN | 6 |
| 4 | RBD: 0.25 mg/mL + CDA: 0.05 mg/mL | RBD: 12.5 | 50 | IN | 8 |
| | | CDA: 2.5 | | | |
| 5 | RBD: 0.25 mg/mL + CDA: 0.1 mg/mL | RBD: 12.5 | 50 | IN | 8 |
| | | CDA: 5 | | | |
| 6 | RBD: 0.25 mg/mL + CDA: 0.15 mg/mL | RBD: 12.5 | 50 | IN | 8 |
| | | CDA: 7.5 | | | |
| 7 | RBD: 0.25 mg/mL + CDA: 0.2 mg/mL | CDA: 10 | 50 | IN | 8 |
| 8 | RBD: 0.25 mg/mL + CDA: 0.05 mg/mL + HBcAg: 0.2 mg/mL | RBD: 12.5 | 50 | IN | 8 |
| | | CDA: 2.5 | | | |
| | | HBcAg: 10 | | | |
| **9** | RBD: 0.25 mg/mL + CDA: 0.1 mg/mL + HBcAg: 0.2 mg/mL | RBD: 12.5 | 50 | IN | 8 |
| | | CDA: 5 | | | |
| | | HBcAg: 10 | | | |
| **10** | RBD: 0.25 mg/mL + CDA: 0.15 mg/mL + HBcAg: 0.2 mg/mL | RBD: 12.5 | 50 | IN | 8 |
| | | CDA: 7.5 | | | |
| | | HBcAg: 10 | | | |
| **11** | RBD: 0.25 mg/mL + CDA: 0.2 mg/mL + HBcAg: 0.2 mg/mL | RBD: 12.5 | 50 | IN | 8 |
| | | CDA: 10 | | | |
| | | HBcAg: 10 | | | |
| **12** | RBD: 0.125 mg/mL + Al(OH)₃: 0.5 mg/mL | RBD: 12.5 | 100 | IM | 8 |
| | | Al(OH)₃: 50 | | | |
| **13** | RBD: 0.125 mg/mL + Al(OH)₃: 0.5 mg/mL + CDA: 0.1 mg/mL | RBD: 12.5 | 100 | IM | 8 |
| | | Al(OH)₃: 50 | | | |
| | | CDA: 10 | | | |

Only with the inclusion of CDA in RBD formulations by the IN route, the anti-RBD antibodies are detected, and only with the effect of CDA the Ab response have the avidity enough to inhibit the interaction of RBD with its ACE2 receptor (Fig. 8) and neutralize the virus *in vitro.* The analysis of the optimal dose evidenced that the use of 10 µg of CDA per mice was optimal when the antigen was administered in similar proportions (10µg of CDA and 12.5µg of RBD) while the dose of 5µg of CDA induced the strongest immune response when the RBD antigen was administered at 50 µg dose (1mg/mL), a surprising characteristic of the novel composition, as shown before. In this sense, we could consider the preferential ranges of concentrations for the RBD/CDA formulations as described in the present application: from RBD: 0.25 mg/mL / CDA: 0.2 mg/mL up to RBD: 1.0 mg/mL / CDA: 0.1 mg/mL and in the case of CDA/RBD/HBcAg formulations adding HBcAg up to 0.2 mg/mL.

In summary, the presented results further confirm that CDA is required to (A) subvert the non-responsiveness to the Pichia derived RBD antigen via IN route, as well as to (B) induce a high intensity / avidity Ab response and also to induce a (C) functional Ab response considering the effect on inhibition of the ACE2-RBD interaction and the viral neutralization. To achieve a robust or even detectable anti-RBD antibody response after intranasal administration the presence of CDA was required. The antibody response induced with the formulations subject matter of the present invention are functional in terms of inhibitory effect of the RBD-ACE2 interaction and in terms of suppression of viral replication in the in vitro models of viral neutralization. The biological response modifier effect of CDA produce an effect on the antigen recipient resulting in the increased avidity of the induced response as previously shown.

### Example 14. CDA and CDA/HBcAg BRM formulations as anti-infective agents.

*In vitro* stimulation of cells using CDA or their combinations with HBcAg has shown an enhancement of different channels of stimulatory effects and a synergistic effect *in vivo* in terms of antiviral type I and immunomodulatory type 2 IFN induction, resulting in the *in vitro* elimination of multiple viruses confirming that combined formulations of both BRMs can be used in the post exposure prophylaxis or early therapy of aged and immunosuppressed individuals, considering the direct antiviral effect due to their enhanced interferon-inducing capacity.

The formulations of the BRMs CDA and HBcAg have been studied according to Table 4 description. Antigen X represents the antigens from viruses causing acute (SARS, IAV, VSR, RV, Dengue) and chronic infections (HBV, HIV, HCV, HPV) in a range of doses from 10 to 200 micrograms, in all cases formulated in phosphate buffered saline excipients. The studied antigens covered the range of inactivated pathogens (as for example the inactivated viruses above described) as well as the subunit antigens of some of these viruses, for example the surface antigen of the hepatitis B, preferably those produced in *Pichia pastoris* or *E*. *coli* host cells. In general, the avidity of the Ab response as well as their functional capacity to neutralize or inhibit the interaction to their target cell receptors has proven superior in the case of formulations described from G3 to G8. The use of multiple agonists of the innate immune system receptors, synergistic enhancers of the IFN responses has potential in post exposure prophylaxis of multiple respiratory pathogens, with special interest in the pandemic sceneries where multiple respiratory pathogens are affecting humans or other animal species.

**Table 4. Description of the formulations studied in vivo and their schedules of administration**

| **Group** | **Formulations (Concentration of the components dissolved in PBS)** | **Schedule of Treatment 1 (route)** | **Schedule of treatment 2 (route)** |
|---|---|---|---|
| **1** | CDA: 0.1mg/mL | Days 1-7 (IN, SL or combined) | 10 dosis every 1/2 weeks (IN) |
| **2** | CDA: 0.2 mg/mL | Days 1-7 (IN, SL or combined) | 10 dosis every 1/2 weeks (IN) |
| **3** | CDA: 0.1 mg/mL + HBcAg: 0.1 mg/mL | Days 1-7 (IN, SL or combined) | 10 dosis every 1/2 weeks (IN) |
| **4** | CDA: 0.2 mg/mL + HBcAg: 0.2 mg/mL | Days 1-7 (IN, SL or combined) | 10 dosis every 1/2 weeks (IN) |
| **5** | CDA: 0.1 mg/mL + HBcAg: 0.1 mg/mL + Ag X | Days 1-7 (IN, SL or combined) | 10 dosis every 1/2 weeks (IN) |
| **6** | CDA: 0.2 mg/mL + HBcAg: 0.2 mg/mL + Ag X | Days 1-7 (IN, SL or combined) | 10 dosis every 1/2 weeks (IN) |
| **7** | CDA: 0.1 mg/mL + HBcAg: 0.1 mg/mL + Ag X | Days 1-7 (IN, SL or combined) | 10 dosis every 1/2 weeks (IN) |
| **8** | CDA: 0.2 mg/mL + HBcAg: 0.2 mg/mL + Ag X | Days 1-7 (IN, SL or combined) | 10 dosis every 1/2 weeks (IN) |
| **9** | HBcAg: 0.1 mg/mL | Days 1-7 (IN, SL or combined) | 10 dosis every 1/2 weeks (IN) |
| **10** | HBcAg: 0.2 mg/mL | Days 1-7 (IN, SL or combined) | 10 dosis every 1/2 weeks (IN) |
| **11** | Ag X | Days 1-7 (IN, SL or combined) | 10 dosis every 1/2 weeks (IN) |
| **12** | Ag X | Days 1-7 (IN, SL or combined) | 10 dosis every 1/2 weeks (IN) |

### Example 15: The use of CDA abrogates the T cell non-responsiveness of mice immunized with RBD.

In example 1, several vaccine formulations were studied to select the composition with the most attractive immune response for SARS-CoV2 prevention. The receptor-binding domain (RBD) protein was used in combination with another BRM (HBcAg). The protein RBD was obtained as a recombinant glycoprotein in *Pichia pastoris,* with the characteristic manosylated pattern of glycosylation and used as immunogen in example 1.

To explore the effect of CDA and their combination with HBcAg on T cell response after nasal administration, CDA and the hepatitis B core antigen (HBcAg), were also used in formulations with RBD. After the third dose, splenocytes from mice immunized with placebo, CDA, RBD, RBD-CDA and RBD-CDA-HBcAg were studied in their capacity to secrete IFN and other cytokines in response to the stimulation with the homologous RBD (produced in Pichia pastoris and the heterologous antigen (produced in CHO), ConA was used as a positive control and a similar amount of cells were used as negative controls and received no treatment.

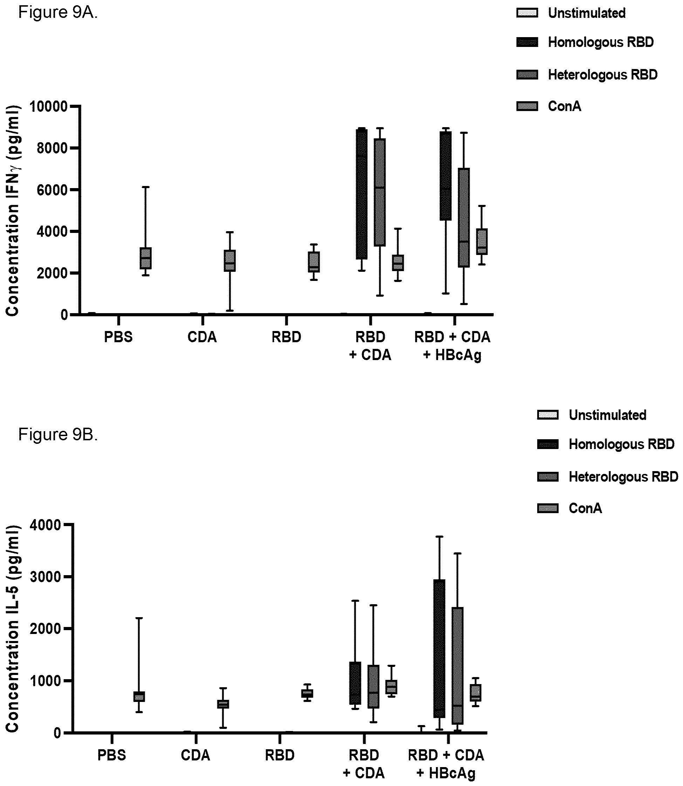

As a result, the nasal administration of RBD in PBS resulted in a non-detectable response of secreted cytokines when the cells were treated with the homologous (Pichia) or heterologous (CHO) RBD antigens. On the other hand, the mice immunized with the RBD antigen in combination with CDA or CDA-HBcAg induced a strong response in terms of secretion of multiple cytokines, at the level of ConA positive control. These results are described in figures 9A and 9B, as representative examples of the different cytokines studied evidencing a mixt Th1/Th2 pattern of response.

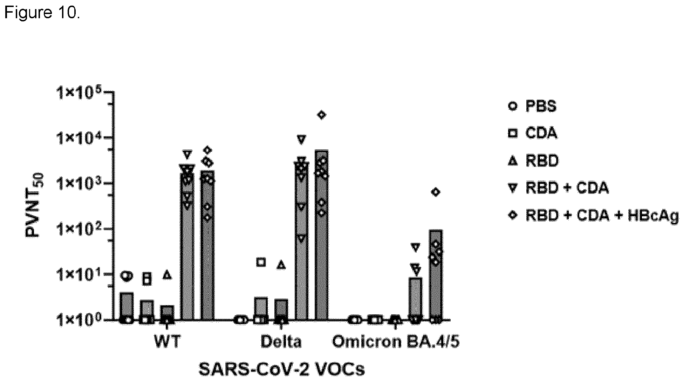

Consistent with the strong Ab response evidenced in examples 1 and 13, and with the T cell response that supported the long-lasting and high avidity of the induced titres, it was detected the capacity of the induced Ab responses to neutralize SARS-CoV-2 from different viral variants, suggesting that the increase of avidity may support the capacity of the induced antibodies to be cross-neutralizing in front of different viral isolates (Fig. 10).

### Example 16: The use of CDA abrogates the T cell non-responsiveness of mice immunized with dengue virus core proteins.

Mice with immune defect in innate immune receptors described in example 1 are unable to generate a detectable T cell immune response specific to the dengue virus core antigens from all 4 serotypes when they receive the antigens administered by IN or IM routes in PBS or alum, respectively. However, the coadministration of CDA in formulation with each of the four core antigens from the 4 distinct serotypes of Dengue virus induced the secretion of high levels of IFN gamma in the supernatant of spleen cells from immunized mice. Similar responses were detected in the multivalent vaccine with the four different antigens administered together and mixed with CDA and alum or by IN route in PBS. The response in the formulations comprising CDA as adjuvant of the monovalent and multivalent composition of dengue virus core antigens was also detected by lymphoproliferative assays both for CD8+ and CD4+ T cells.

## Claims

1. A cyclic dinucleotide (CDN) for use as a biological response modifier for improving the immune response of subject in need thereof with underperforming immune systems **characterized by** low/non-responsiveness to vaccine antigens or underperforming immune response to pathogens.

2. The CDN for use according to claim 1, wherein the CDN is a CDN according to formula I: 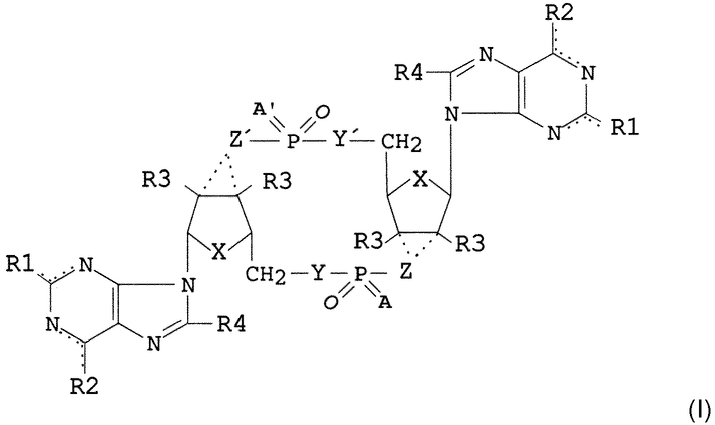 wherein
A, A' is independently from one another S or O;
X is independently from one another S, N, O, CH₂;
Y, Y' is independently from one another NH, CH₂, O;
Z, Z' is independently from one another NH, CH₂, O;
R₁ is independently from one another hydrogen, NH₂,S or O;
R₂ is independently from one another NH₂, O, H, or S;
R₃ is independently from one another absent if a covalent bond is present between the Z or Z' and the C atom, or is hydrogen, OH, halogen, a straight or branched C₁-C₆ alkyl group, or a straight or branched C₁-C₆ alkoxy group which may optionally be substituted;
R₄ is independently from one another hydrogen, halogen, or a straight or branched C₁-C₆ alkyl group, which may optionally be substituted;
... is a single or double bond; or conjugates thereof, and salts or solvates thereof.

3. The CDN for use according to claim 1 or 2 as a biological response modifier in combination with at least one different biological response modifier.

4. The CDN for use according to claim 2 or 3 **characterized in that** is the compound according to formula I any one of the purine residues is an adenine, xanthine or hypoxanthine residue or combinations thereof, in particular, both purine residues being adenine.

5. The CDN for use according to any one of claims 2 to 4 **characterized in that** in formula I R3 is a OH group, X is an oxygen atom and Y, Y', Z and Z' are oxygen.

6. The CDN for use according to any one of claims 2 to 5 **characterized in that** the compound according to formula I is a bis-(3'-5')-cyclic dimeric adenosine monophosphate (CDA or c-di-AMP), c-di-GMP, c-di-IMP, c-di-GAMP, c-IAMP, or a cyclic di-AMP thiophosphate, c-di-IMP thiophosphate and c-IAMP thiophosphate.

7. The CDN for use according to any one of the preceding claims, to generate a fast immune response as part of vaccine compositions administered in accelerated schedules of treatment, specifically weekly, biweekly or monthly administration schedules.

8. The CDN for use according to any one of the preceding claims to improve an immune response by boosting at the time of contraction of the germinal center reaction to further foster antibody hypersomatic mutation and affinity maturation.

9. The CDN for use according to any one of the preceding claims, wherein the CDN is administered by mucosal route, in particular, wherein the mucosal route is selected from intranasal, sublingual, intra-tracheal or pulmonary.

10. The CDN for use according to any one of claims 1 to 9, wherein the CDA is administered parenterally, in particular, intramuscularly, subcutaneous, intradermal and transcutaneous or perifollicular.

11. The CDN for use according to any one of the preceding claims, wherein the CDN is administered as biological response modifier as part of a prime-boost or prime-pull scheme of vaccination.

12. The CDN for use according to any one of claims 1 to 11, (i) in the setting of epidemic or pandemic immunostimulation where the biological response modifier is administered alone or in combination with other biological response modifier in a pre-/post exposure prophylaxis setting in absence of any vaccine antigen, or (ii) in formulation with the antigens of an infectious agent in the group of chronic infectious diseases, or (iii) in formulation with the antigens of pathogens from the group of acute respiratory and/or febrile infections.

13. The CDN for use according to any one of claims 1 to 11, in vaccine formulations comprising one or several antigens from viruses, like coronavirus, the antigens of SARS-CoV-2.

14. The CDN for use according to any one of claims 1 to 11, in vaccine formulations comprising the antigen S (Spike) of coronavirus or the subunit antigen RBD.

15. The CDN for use according to any one of the preceding claims, to immunize an individual with a reduced capacity of response to RNA viruses and variants thereof, such as SARS-CoV-2 or other beta-coronaviruses, due to immune senescence or immunodepression, particularly, in combinations with other biological response modifiers capable of further improving the effect of the CDN, allowing dose splitting or adding complementary properties to the resulting formulation, like wherein the other biological response modifier corresponds to the Hepatitis B nucleocapsid antigen or core, with a multi-TLR agonist complementary effect to split the effective dose and to further stimulate the innate immune system and non-specific immunity, of value in immune compromised or vulnerable patients, or the elderly or immune senescent population.

16. The formulations of a biological response modifier according to any of the precedent claims wherein the preferred CDN dose correspond to the range between 0.01 and 0.4 mg per dose, and the formula may comprise, in addition, HBcAg in a similar preferred range and may also contain a selected antigen in a dose range from 0.01 to 0.2 mg, in an excipient such as but not limited to phosphate buffered saline solution.
